# EUROPEAN PATENT APPLICATION

(11) **EP 0 716 084 A1**
(43) Date of publication of application: **12.06.1996**
(21) Application number: 95111007.1
(22) Date of filing: 13.07.1995
(51) Int. Cl.: C07D 295/08

(54) **Preparation of 4,4'-(oxydi-2,1-ethanediyl)bis-morpholine and hydroxyethoxyethyl morpholine**

(30) Priority: 09.12.1994 US 352649
(71) Applicant: HUNTSMAN CORPORATION, Austin, Texas 78752 (US)
(72) Inventor: Zimmerman, Robert LeRoy, Austin, Texas 78759 (US)
(74) Representative: Goddar, Heinz J., Dr.

(57) **Abstract**

This invention discloses a process comprising reacting diethylene glycol and morpholine in the presence of hydrogen over a catalyst selected from the group consisting of copper; cobalt on amorphous silica; and cobalt on alumina to produce compounds having the formulae: 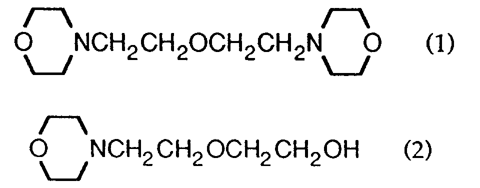
This invention also discloses a process comprising recycling product compound (2) into the same reaction to increase the yield to compound (1). Compound (1) is known by the names 4,4'-(oxydi-2,1-ethanediyl)*bis*-morpholine; *bis*(morpholinoethyl)ether; N,N',2,2'-dimorpholinodiethylether or TEXACAT® DMDEE. Compound (2) is known by the names 4-(2(2-hydroxyethoxy)ethyl) morpholine and hydroxyethoxyethyl morpholine (HEEM).

## Description

### Field of the Invention

This invention discloses the preparation of 4,4'-(oxydi-2,1-ethanediyl)*bis*-morpholine, also referred to as *bis*(morpholinoethyl)ether; N,N',2,2'-dimorpholinodiethylether or TEXACAT® DMDEE (DMDEE) together with 4-(2(2-hydroxyethoxy)ethyl) morpholine also referred to as hydroxyethoxyethyl morpholine (HEEM). Synthesis is from diethylene glycol and morpholine in the presence of hydrogen over a copper or cobalt catalyst on amorphous silica or alumina gel.

### Background of the Invention

In prior art processes the typical starting material for the product of the instant invention is hydroxyethyl morpholine, a reaction product of ethylene oxide and morpholine.

U.S. Patent 3,087,928 discloses a method for preparing N-alkylmorpholine by reacting a morpholine compound with an alkanol in the presence of hydrogen and a catalyst comprising cobalt or nickel, copper and a promoter selected from the group of chromium oxide, titanium oxide, thorium oxide, magnesium oxide, zinc oxide, manganese oxide and rare earth oxides.

U.S. Patent 4,582,904 discloses the synthesis of organic amines by condensation of organic hydroxy compound with ammonia or a primary or secondary amine in the presence of a rare earth metal hydrogen phosphate catalyst.

U.S. Patent 4,103,087 discloses the preparation of a di-(N,N-disubstituted amino)alkane compound by reacting an (N,N-disubstituted) amino alkanol compound with an (N,N-disubstituted) amine compound in the presence of an aluminum phosphate catalyst at a temperature of from about 240°C to 320°C.

Canadian Patent 1,078,382 discloses a process for selectively producing a *bis*(N,N-disubstituted amino) compound which includes contacting an (N,N-disubstituted) amine compound with a diol compound in the presence of a phosphorous-containing catalyst.

U.S. Patent 4,117,227 discloses a process for selectively producing an N-(substituted) morpholine compound from the corresponding N-(substituted) diethanolamine compound utilizing a silica-alumina or phosphorous containing catalyst, *e.g.* ferric phosphate.

U.S. Patent 3,817,997 discloses a process whereby 2,2'-*bis*(4-morpholino)diethyl ether, a polyurethane catalyst, is formed by contacting amine residues with a hydrogenation catalyst (which may include Ni, Cu, Fe, Pd, Pt, Rh, Co, Cr, Mn, Ti, Mo, V, Ru, etc.) at a temperature within the range of about 180° to 100°C and a pressure with the range of about 200 to 10,000 psig. The amine residue is obtained by reacting diethylene glycol with ammonia at a temperature within the range of about 150° to 400°C and a pressure in the range of about 450 to 6,000 psig in the presence of a hydrogenation catalyst.

I. G. Kronich, et al., "Gas-phase Synthesis of Morpholine from Diethylene Glycol and Ammonia", *Khim. Prom-st*. *(The Soviet Chemical Industry)*, Vol. 14, No. 11, 1982, pp, 653-655, discloses the production of 2,2'-di(N-morpholino)diethyl ether by the reaction of diethylene glycol with morpholine in the presence of ammonia and hydrogen using a nickel catalyst promoted with copper and chromium. These authors find this reaction to be reversible using this catalyst. Interestingly, in the process of producing morpholine from diethylene glycol and ammonia, nickel, cobalt and copper catalysts were tried. Only the catalysts containing nickel were found to have high activity for this reaction. The cobalt catalysts had low activity and the copper ones were practically inactive.

U.S. Pat. 4,026,935 describes a process for selectively producing a *bis*-(morpholino-N-alkyl) ether directly from the corresponding N-(hydroxyalkyl) morpholine compound. The improved process includes contacting the N-(hydroxyalkyl) morpholine compound with a catalytically effective amount of a silica-alumina or certain phosphorus-containing substances at a temperature of about 200°C to 300°C under a pressure sufficient to maintain the mixture substantially in liquid phase and recovering from the resultant reaction mixture the *bis*-(morpholino-N-alkyl) ether.

It would be further desirable if a process for making these materials in high selectivities, high conversions and high purities. The prior art processes produce DMDEE in relatively low selectivities, low purities and poor color, such as yellow to orange-brown in color.

### Summary of the Invention

Accordingly, it is an object of the present invention to provide a process for making DMDEE and HEEM from morpholine and diethylene glycol (DEG) in high selectivity.

It is another object of the present invention to provide a process for producing DMDEE in high purity and of water white clarity.

In carrying out these and other objects of the invention, there is provided, in one form, a process comprising reacting diethylene glycol and morpholine, in the presence of hydrogen and a catalyst selected from the group consisting of copper, cobalt on amorphous silica, and cobalt on alumina, to produce 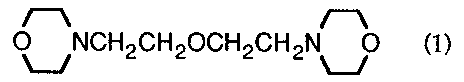
4,4'-(oxydi-2,1-ethanediyl)*bis*-morpholine and 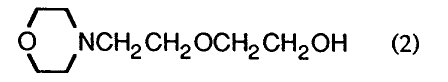
4-(2(2-hydroxyethoxy)ethyl) morpholine. The inventive process optionally includes recycling product (2) of the reaction above into the same reaction to increase production of product (1).

### Description of the Preferred Embodiment

This invention is the reaction of diethylene glycol (DEG) and morpholine in the presence of hydrogen over a copper or cobalt catalyst on amorphous silica or alumina gel to produce 4,4'-(oxydi-2,1-ethanediyl)*bis*-morpholine also referred to as *bis*(morpholinoethyl)ether; N,N',2,2'-dimorpholinodiethylether or TEXACAT® DMDEE (hereinafter referred to as DMDEE) together with 4-(2(2-hydroxyethoxy)ethyl) morpholine also referred to as hydroxyethoxyethyl morpholine (hereinafter referred to as HEEM). The reaction may be schematically illustrated as shown below: 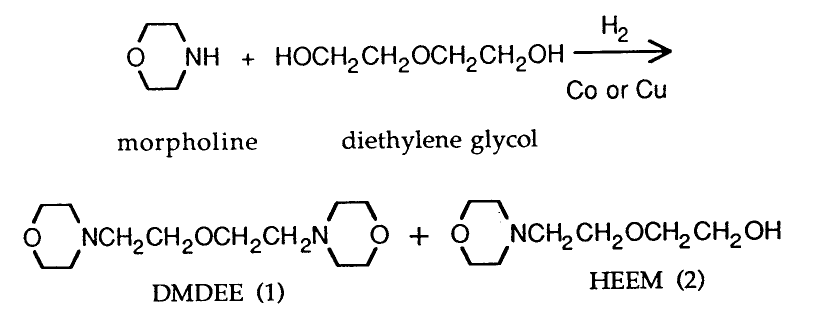
The copper catalyst may also optionally contain zinc, magnesium, or mixtures thereof. The preferred copper catalyst is a copper-zinc catalyst. No other promoters are used. The preferred catalyst for the instant invention is a cobalt on amorphous silica catalyst that gives much better selectivity to the combination of DMDEE and HEEM than the nickel-copper-chromium or cobalt-copper-chromium catalysts of U.S. Pat. Nos. 3,087,928 and 3,817,997, incorporated herein by reference. No promoters are used in conjunction with the cobalt, whether supported on silica or alumina.

The reactions may be carried out at from about 150°C to about 250°C and from about 0 psig to about 5000 psig. The preferred temperature range is from about 210°C to about 230°C. The preferred pressure is about 1000 to about 2500 psig. The reactions of this invention may be carried out in batch or tubular reactors. The preferred reactor is a tubular reactor so that the reaction may be conducted continuously. Hydrogen should be present in the reaction, but no ammonia is added.

An advantage to this reaction is that there is a higher selectivity to the overall production of HEEM and DMDEE by the recycling of HEEM through the reaction to fully convert it to DMDEE, thus producing a higher total conversion of the preferred product DMDEE. It is apparent from the stoichiometry of the reaction schematically illustrated above that one mole of morpholine reacts with one mole of DEG to give HEEM, while two moles of morpholine reacts with one mole of DEG to give DMDEE. Further, one mole of morpholine may react with one mole of HEEM to give DMDEE, in the recycle step of this invention.

The weight ratio of morpholine to DEG may range from about 1.4/1 to about 10/1.

The method of this invention unexpectedly achieves a combined selectivity to DMDEE and HEEM of at least 85%, preferably at least 90%. A further improvement of the method of preparation outlined in this invention is that the product TEXACAT® DMDEE is obtained at about 99% purity and is water white. With other processes, neither the purity nor the lack of color are obtained.

The following Examples are merely illustrative and should not be construed as limitations on the scope of the claims.

### EXAMPLES 1-18

In the following Examples shown in Table I, a tubular reactor was used. Diethylene glycol, morpholine and hydrogen were fed to the reactor. The pressure of the reactor for all reactions was 2000 psig. The reaction effluent was analyzed by gas chromatograph. The feed rates of morpholine and diethylene glycol, reaction temperatures and catalysts used are given in Table I. The hydrogen feed rate for all reactions was 27 l/hr. The morpholine/diethylene glycol weight percent ratio can be calculated by dividing the feed rate of morpholine by the feed rate of diethylene glycol, both in grams per cubic centimeter of catalyst per hour (g/cc-cat-hr).

The following examples compare the prior art catalysts of U.S. Pat. Nos. 3,087,928 and 3,817,997 to the catalysts of this invention. These prior art catalysts include Ni-Cu-Cr and Co-Cu-Cr. The catalysts of this invention include a copper catalyst containing zinc and a cobalt catalyst on an amorphous silica support.

As can be seen in Table I, the catalysts of this invention show a much better selectivity to HEEM and DMDEE than do the prior art catalysts. The comparison also teaches that cobalt on amorphous silica catalyst has a better selectivity to DMDEE than does copper with zinc catalyst. Moreover, the Cu and Co catalysts of this invention produce products that are water white; whereas, the prior art catalysts, *e.g*. those in U.S. Pat. No. 4,026,935, incorporated by reference herein, produce products that are yellow to orange brown in color. Thus, the catalysts of the inventive process provide a higher selectivity to the desired product without product discoloration.

Both the copper-zinc catalyst and the cobalt on amorphous silica gave 84% or better conversion to DMDEE and HEEM. Most were over 85% and many were over 90%. The prior art Ni-Cu-Cr catalyst and the Co-Cu-Cr catalyst gave 77% as their best selectivity. Many of the selectivities for the latter catalysts were in the 50% to 60% range.

As example 18 shows, both high conversion of DEG (92%) and good selectivity to DMDEE (72%) can be achieved with cobalt on amorphous active. With other catalysts both cannot be obtained simultaneously. Distillation of a run like Example 18 gave 99.7% pure DMDEE that was water white.

**Table I**

| Production of DMDEE and HEEM | | | | | | | | |
|---|---|---|---|---|---|---|---|---|
| Ex. | Catalyst | DEG (g/cc-cat-hr) | Morpholine (g/cc-cat-hr) | °C | Selectivity | | | Conversion DEG % |
| | | | | | DMDEE % | HEEM % | HEEM + DMDEE % | |
| 1 | Ni-Cu-Cr | 0.24 | 0.50 | 210 | 52 | 11 | 63 | 97 |
| 2 | " | " | " | 220 | 46 | 7 | 53 | 97 |
| 3 | " | " | " | 230 | 45 | 6 | 51 | 96 |
| 4 | Co-Cu-Cr | 0.28 | 0.85 | 200 | 23 | 47 | 70 | 59 |
| 5 | " | " | " | 210 | 32 | 35 | 67 | 82 |
| 6 | " | " | " | 220 | 34 | 43 | 77 | 82 |
| 7 | " | " | " | 230 | 44 | 23 | 67 | 100 |
| 8 | Cu-Zn | 0.24 | 0.73 | 210 | 7 | 83 | 90 | 25 |
| 9 | " | " | " | 220 | 20 | 71 | 91 | 56 |
| 10 | " | " | " | 230 | 40 | 50 | 90 | 85 |
| 11 | Co/amorphous silica | 0.24 | 0.73 | 200 | 30 | 62 | 92 | 43 |
| 12 | " | " | " | 210 | 48 | 43 | 91 | 53 |
| 13 | " | " | " | 220 | 59 | 30 | 89 | 75 |
| 14 | " | " | " | 230 | 66 | 18 | 84 | 89 |
| 15 | " | 0.25 | 0.50 | 200 | 36 | 29 | 95 | 48 |
| 16 | " | " | " | 210 | 53 | 40 | 93 | 72 |
| 17 | " | " | " | 220 | 66 | 27 | 93 | 81 |
| 18 | " | " | " | 230 | 72 | 18 | 90 | 92 |

Many modifications may be made in the process of this invention without departing from the spirit and scope thereof which are defined only in the appended claims. For example, one skilled in the art may discover that a certain combination or proportion of reactants and operating conditions give particularly optimal results. Alternatively, different copper and cobalt catalysts within the claim definitions may prove advantageous.

The features disclosed in the foregoing description and in the following claims may, both separately and in any combination thereof, be material for realising the invention in diverse forms thereof.

## Claims

1. A process comprising reacting diethylene glycol and morpholine, in the presence of hydrogen and a catalyst selected from the group consisting of copper, cobalt on amorphous silica, and cobalt on alumina, to produce 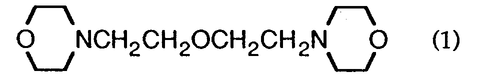 4,4'-(oxydi-2,1-ethanediyl)*bis*-morpholine and 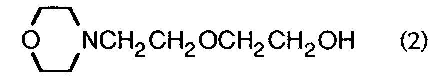 4-(2(2-hydroxyethoxy)ethyl) morpholine.

2. The process of claim 1 where the reacting is carried out at a temperature from about 150°C to about 250°C and a pressure of from about 0 psig to about 5000 psig.

3. The process of claim 2 where the catalyst is a cobalt catalyst on amorphous silica and the process is conducted continuously.

4. The process of claim 1 where compound (2) is recycled to increase yield to compound (1).

5. The process of claim 1 in where the catalyst is a copper catalyst further comprising zinc.

6. The process of claim 1 where the selectivity to the combination of compounds (1) and (2) is at least 85%.

7. A process for producing comprising 4,4'-(oxydi-2,1-ethanediyl)*bis*-morpholine having the formula: 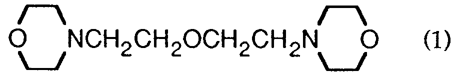 comprising the steps of:
reacting diethylene glycol and morpholine, in the presence of hydrogen and a catalyst selected from the group consisting of copper, cobalt on amorphous silica, and cobalt on alumina, at a temperature from about 150°C to about 250°C and a pressure of from about 0 psig to about 5000 psig, to produce a mixture of compound (1) and 4-(2(2-hydroxyethoxy)ethyl) morpholine having the formula: 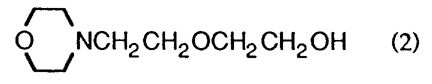 ; and
recycling compound (2) to the reacting to increase the yield to compound (1).

8. The process of claim 7 where the catalyst is a cobalt catalyst on amorphous silica and the process is conducted continuously.

9. The process of claim 7 in where the catalyst is a copper catalyst further comprising zinc.

10. The process of claim 1 where the selectivity to the combination of compounds (1) and (2) in the reacting is at least 85%.

11. A process for producing comprising 4,4'-(oxydi-2,1-ethanediyl)*bis*-morpholine having the formula: 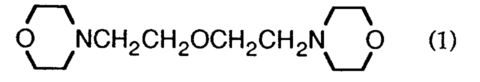 comprising the steps of:
reacting diethylene glycol and morpholine, in the presence of hydrogen and a cobalt catalyst on amorphous silica at a temperature from about 150°C to about 250°C and a pressure of from about 0 psig to about 5000 psig, to produce a mixture of compound (1) and 4-(2(2-hydroxyethoxy)ethyl) morpholine having the formula: 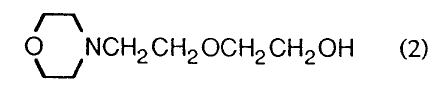 where the selectivity to the combination of compounds (1) and (2) is at least 85%; and
recycling compound (2) to the reacting to increase the yield to compound (1).

12. The process of claim 11 where the process is conducted continuously.
